# EUROPÄISCHE PATENTSCHRIFT

(11) **EP 2 806 944 B1**
(45) Veröffentlichungstag und Bekanntmachung des Hinweises auf die Patenterteilung: **21.09.2016**
(21) Anmeldenummer: 13701608.5
(22) Anmeldetag: 22.01.2013
(51) Int. Cl.: A61N 1/372

(54) **TRAGEVORRICHTUNG ZUM TRAGEN EINER SENDESPULE AM KÖRPER EINES PATIENTEN**
SUPPORT DEVICE FOR SUPPORTING A TRANSMISSION COIL ON THE BODY OF A PATIENT
DISPOSITIF DE SUPPORT POUR SUPPORTER UNE BOBINE D'ÉMISSION SUR LE CORPS D'UN PATIENT

(30) Priorität: 25.01.2012 DE 102012201073
(43) Veröffentlichungstag der Anmeldung: 03.12.2014
(73) Patentinhaber: Dualis Medtech GmbH, 82229 Seefeld (DE)
(72) Erfinder: SCHMID, Thomas, 86938 Schondorf (DE); AURACHER, Stefan, 82211 Herrsching (DE); SCHWARZBACH, Stefan, 82205 Gilching (DE)
(74) Vertreter: dompatent von Kreisler Selting Werner - Partnerschaft von Patent- und Rechtsanwälten mbB
(86) Internationale Anmeldenummer: PCT/EP2013/051131
(87) Internationale Veröffentlichungsnummer: WO 2013/110602

(56) Entgegenhaltungen:
- US-A1- 2005 192 644
- US-A1- 2007 123 948
- US-A1- 2010 114 143

## Beschreibung

Die Erfindung betrifft ein System mit einer Tragevorrichtung zum Tragen einer Sendespule am Körper eines Patienten.

Es ist bekannt, Implantate, beispielsweise Kunstherzen oder Herzunterstützungssysteme, durch eine induktive Kopplung unter Verwendung einer implantierten Empfangsspule und einer extrakorporalen Sendespule drahtlos mit Energie zu versorgen. Hierzu wird die Sendespule mit einer Spannungsquelle verbunden, sodass ihr eine elektrische Wechselspannung zugeführt wird, die ein alternierendes Magnetfeld erzeugt. Die Empfangsspule, die im Bereich dieses Magnetfeldes angeordnet ist, erzeugt durch Induktion eine elektrische Spannung, die dem Implantat zugeführt wird.

Um eine Energieübertragung mit einem hohen Wirkungsgrad zu erzielen, ist es wichtig, die Sendespule exakt über der implantierten Empfangsspule anzuordnen. Ein Versatz zwischen der Sendespule und der Empfangsspule, sowohl in radialer als auch in axialer Richtung, verschlechtert den Wirkungsgrad der Energieübertragung. Ferner sollte die Tragevorrichtung, durch die die Sendespule am Körper des Patienten gehalten wird, in komfortabler Weise am Körper des Patienten getragen werden können und diesen möglichst wenig in seinen täglichen Aktivitäten beeinträchtigen.

Es ist bekannt, die Sendespule durch eine Befestigungs- oder Tragevorrichtung am Körper des Patienten zu befestigen, sodass diese induktiv mit der implantierten Empfangsspule gekoppelt werden kann. Eine solche Tragevorrichtung kann beispielsweise in Form eines Gürtels ausgebildet sein. Es besteht das Problem, dass eine Energieübertragung nicht mehr gewährleistet werden kann, wenn eine solche Tragevorrichtung verrutscht und somit die Sendespule nicht mehr exakt über der Empfangsspule angeordnet ist. Weiterhin kann durch äußere Einwirkungen ein unbeabsichtigtes Ablegen einer solchen Tragevorrichtung erfolgen, sodass in diesem Fall ebenfalls keine Energie mehr übertragen werden kann. Derartige Szenarien können zu gefährlichen, teilweise lebensbedrohlichen Situationen führen.

Aufgabe der Erfindung ist es, ein System mit einer Tragevorrichtung zum Tragen einer Sendespule am Körper eines Patienten zu schaffen, mit der eine zuverlässigere Energieübertragung an eine implantierte Empfangsspule erreicht werden kann. Die Lösung der Aufgabe erfolgt erfindungsgemäß durch die Merkmale der Ansprüche 1 und 6.

Gemäß einer besonderen Ausführungsform der Erfindung kann die Sendespule durch elektrisch leitende Elemente, beispielsweise Litzen, ausgebildet sein, die in die Tragevorrichtung eingewebt und spulenförmig gewickelt sind, wodurch ein besonders hoher Tragekomfort erreicht werden kann.

Die erfindungsgemäße Tragevorrichtung kann verschiedene Formen haben und beispielsweise als Weste, Gürtel oder Bustier ausgebildet sein. Die erfindungsgemäße Tragevorrichtung umfasst eine Detektionsvorrichtung zum Erkennen eines Anlegevorgangs, eines Anlegezustandes, eines Ablegevorgangs und/oder eines Ablegezustandes der Tragevorrichtung relativ zum Körper des Patienten. Die Detektionsvorrichtung ist somit in der Lage, mindestens eine der genannten Handlungen oder Zustände zu erkennen, nämlich, ob die Tragevorrichtung gerade am Körper des Patienten angelegt wird, ob die Tragevorrichtung am Körper des Patienten angelegt ist, ob die Tragevorrichtung gerade vom Körper des Patienten abgelegt wird oder ob die Tragevorrichtung zum gegenwärtigen Zeitpunkt bereits abgelegt ist und sich somit nicht mehr am Körper des Patienten befindet.

Durch die genannte Detektionsvorrichtung steht der erfindungsgemäßen Tragevorrichtung somit die wichtige Information zur Verfügung, ob die Tragevorrichtung in korrekter Weise am Körper des Patienten angebracht ist und somit eine ordnungsgemäße Energieübertragung erfolgen kann. Sofern dies nicht der Fall ist, können geeignete Maßnahmen eingeleitet werden, die im Folgenden beschrieben werden. Durch die Erfindung wurde somit eine intelligente Tragevorrichtung geschaffen, durch die gefährlichen und möglicherweise lebensbedrohlichen Situationen vorgebeugt werden kann, da sie Informationen über den Tragezustand berücksichtigt, die vorher nicht zur Verfügung standen.

In einer bevorzugten Ausführungsform weist die Tragevorrichtung eine Verschlussvorrichtung zum lösbaren Befestigen der Tragevorrichtung am Körper des Patienten auf. Hierbei kann es sich beispielsweise um einen Reißverschluss, einen oder mehrerer Klett- oder Schnappverschlüsse oder ähnliche Verschlussvorrichtungen handeln. Die Detektionsvorrichtung kann in oder an der Verschlussvorrichtung derart ausgebildet sein, dass durch die Detektionsvorrichtung ein Öffnen der Verschlussvorrichtung und bei mehreren Verschlussvorrichtungen, insbesondere der zeitliche Verlauf des Öffnungsvorgangs der mehreren Verschlussvorrichtungen erfassbar ist. Somit steht der erfindungsgemäßen Tragevorrichtung die Information zur Verfügung, ob die Verschlussvorrichtung geöffnet wurde, sowie gegebenenfalls die Art oder der zeitliche Verlauf des Öffnungsvorgangs. Aus dem zeitlichen Verlauf des Öffnungsvorgangs können Informationen darüber gewonnen werden, ob die Verschlussvorrichtung beabsichtigt oder unbeabsichtigt geöffnet wurde. Weitere Details hierzu werden im Zusammenhang mit dem erfindungsgemäßen Verfahren im weiteren Verlauf der vorliegenden Anmeldung beschrieben.

Es ist beispielsweise möglich, jeweils eine Detektionsvorrichtung an jeweils einer Verschlussvorrichtung vorzusehen, sodass detektierbar ist, wann welche Verschlussvorrichtung geöffnet wurde. Weiterhin können entlang einer einzigen Verschlussvorrichtung, beispielsweise entlang eines Reißverschlusses, mehrere Detektionsvorrichtungen angeordnet sein, sodass erfasst werden kann, auf welche Weise und in welcher Geschwindigkeit der Reißverschluss geöffnet wurde. Wird der Reißverschluss beispielsweise nicht in der vorgesehenen Richtung, zum Beispiel von oben nach unten geöffnet, so kann davon ausgegangen werden, dass er durch eine äußere Krafteinwirkung unbeabsichtigt geöffnet wurde und somit das anschließende Ablegen der Tragevorrichtung nicht beabsichtigt war. In diesem Fall kann ein entsprechender Alarm ausgegeben werden oder es können weitere Gegenmaßnahmen eingeleitet werden. Weiterhin ist es zum Beispiel möglich, dass eine Verschlussvorrichtung, beispielsweise ein Reißverschluss, während des Schlafs des Patienten durch Bewegungen des Patienten unbeabsichtigt geöffnet wird. Dies geschieht üblicherweise über einen relativ langen Zeitraum. Wird somit durch die Detektionsvorrichtungen ein Öffnen der Verschlussvorrichtung oder der Verschlussvorrichtungen über einen Zeitraum detektiert, der länger als ein definierter Schwellwert ist, so kann von einem unbeabsichtigten Öffnen der Verschlussvorrichtung ausgegangen werden. Weiterhin kann die Information von Interesse sein, in welchem Ausmaß eine Verschlussvorrichtung durch den Patienten geschlossen wurde. Zur Erfassung dieser Information können beispielsweise entlang der Verschlussvorrichtung oder an mehreren Verschlussvorrichtungen mehrere Detektionsvorrichtungen angeordnet sein, durch die erfasst wird, ob die Verschlussvorrichtung an diesem Punkt geschlossen ist. Beispielsweise kann hierdurch erfasst werden, ob ein Reißverschluss durch den Patienten vollständig geschlossen wurde. Sofern dies nicht der Fall ist, wird davon ausgegangen, dass die Sendespule möglicherweise nicht richtig positioniert ist, so dass ein Alarm ausgegeben wird.

Die erfindungsgemäße Tragevorrichtung weist ferner eine Eingabevorrichtung zum Eingeben einer Information über ein beabsichtigtes Ablegen der Tragevorrichtung durch den Benutzer in die Tragevorrichtung auf. Der Benutzer kann somit über diese Eingabevorrichtung der Tragevorrichtung die Information zur Verfügung stellen, dass er die Tragevorrichtung beabsichtigt abgelegt hat oder ablegen wird. Bei der Eingabevorrichtung kann es sich beispielsweise um eine Spracheingabevorrichtung, zum Beispiel ein Mikrofon, oder mindestens eine Taste handeln. Hierbei kann es sich auch um eine Softwaretaste auf einem Touchdisplay handeln, die nicht als Hardwaretaste ausgebildet ist. Die Tragevorrichtung weist weiterhin eine Alarmvorrichtung zum Ausgeben eines Alarmsignals bei einem unbeabsichtigten Ablegen oder Verschieben der Tragevorrichtung auf. Alternativ oder zusätzlich kann die Tragevorrichtung mit einer solchen Alarmvorrichtung datentechnisch verbindbar sein, sodass es möglich ist, den Alarm an einer anderen Stelle auszugeben. Beispielsweise kann der Alarm auch an eine medizinische Institution weitergeleitet werden, sodass ein Arzt, Sanitäter oder andere Hilfskräfte bei einem unbeabsichtigten Ablegen der Tragevorrichtung entsprechende Hilfsmaßnahmen einleiten können. Eine solche Übermittlung des Alarmsignals kann auch über größere Entfernungen erfolgen, indem sie beispielsweise über ein insbesondere drahtloses Kommunikationsnetzwerk erfolgt. Ein Alarmsignal oder andere Informationen über den Tragezustand oder über den Gesundheitszustand des Patienten können auch an eine andere elektronische Vorrichtung, beispielsweise ein Smartphone oder eine Uhr, die vom Patienten getragen wird, übermittelt werden. Durch die Alarmvorrichtung erfolgt kein Ausgeben eines Alarmsignals, sofern durch den Benutzer an der Eingabevorrichtung die Eingabe einer Information über ein beabsichtigtes Ablegen der Tragevorrichtung erfolgt ist. Der Alarm wird lediglich dann ausgegeben, wenn die Tragevorrichtung davon ausgeht, dass sie unbeabsichtigt abgelegt wurde. Hierdurch können Fehlalarme vermieden werden, die sowohl beim Patienten als auch bei anderem Hilfspersonal unnötigen Aufwand verursachen.

So ist es beispielsweise dem Benutzer möglich, in einer bestimmten Situation, beispielsweise vor dem Duschen, der Eingabevorrichtung die Information zur Verfügung zu stellen, dass die Tragevorrichtung nun in beabsichtigter Weise abgelegt wird, sodass kein Alarm ausgegeben wird. Während der Zeit, in der die Tragevorrichtung abgelegt ist, wird das Implantat durch einen ebenfalls implantierten Akku mit Energie versorgt. Nach dem Duschen kann der Benutzer die Tragevorrichtung wieder anlegen, sodass die Sende- und Empfangsspule wieder miteinander gekoppelt werden und die induktive Energieübertragung wieder stattfinden kann. Zu diesem Zeitpunkt kann der Patient der Tragevorrichtung eine weitere Information darüber zur Verfügung stellen, dass das beabsichtigte Ablegen der Tragevorrichtung nun beendet ist, sodass bei einem erneuten Ablegen der Tragevorrichtung davon ausgegangen werden kann, das dies unbeabsichtigt erfolgt ist.

Weiterhin kann die Tragevorrichtung einen Sensor zur Erfassung von Bewegungen des Patienten oder der Position des Patienten aufweisen. Hierbei kann es sich beispielsweise um einen GPS-Sensor handeln. Alternativ kann die Position über das GSM-Netz ermittelt werden. Weiterhin kann ein Lagesensor zur Erfassung der Lage des Patienten vorgesehen sein. Über die genannten Sensoren können insbesondere der zeitliche Verlauf der Bewegungen und die Lage des Patienten erfasst werden. Hieraus können wichtige Informationen über den Therapieverlauf gewonnen werden. Weiterhin können diese Informationen für den Hersteller der Tragevorrichtung nützlich sein, da er dadurch die Tragevorrichtung an das Nutzungsverhalten der Patienten anpassen kann. Zum Beispiel kann erfasst werden, wie oft die Tragevorrichtung vom Patienten abgelegt wird. Alternativ oder zusätzlich kann das Ladeverhalten erfasst werden. Dies bedeutet, dass erfasst wird, in welchen zeitlichen Abständen und wie lange der implantierte Akku unter Verwendung der Sendespule in der Tragevorrichtung aufgeladen wird. Hieraus können wichtige Informationen bezüglich der Dimensionierung des Akkus gewonnen werden.

Weiterhin kann die erfindungsgemäße Tragevorrichtung eine Versatzerkennungsvorrichtung zum Erkennen eines axialen oder radialen Versatzes zwischen der Sendespule, die in oder an der Tragevorrichtung angeordnet ist, und einer im Körper des Patienten implantierten Empfangsspule aufweisen. Eine derartige Versatzerkennungsvorrichtung kann beispielsweise durch Magnetfeldsensoren im Bereich der implantierten Empfangsspule ausgebildet sein, die die Stärke und/oder Richtung des von der Sendespule generierten Magnetfeldes erfassen. Alternativ oder zusätzlich können elektrische Parameter, beispielsweise die in der Empfangsspule induzierte Spannung, der Strom oder die Phasenlage, in der Empfangsspule gemessen werden. Aus diesen Parametern kann ebenfalls ein Versatz zwischen Sendespule und Empfangsspule abgeleitet werden. In der genannten Ausführungsform weist die Tragevorrichtung ferner eine Alarmvorrichtung auf, wobei bei einem Versatz, der den definierten Schwellwert überschreitet, ein Ausgeben eines Alarmsignals erfolgt, sofern durch die beschriebene Eingabevorrichtung kein Eingeben einer Information über ein beabsichtigtes Ablegen der Tragevorrichtung durch den Patienten erfolgt ist.

Die beschriebene Versatzerkennungsvorrichtung kann in einer besonderen Ausführungsform auch mit der beschriebenen Detektionsvorrichtung zum Detektieren des Schließzustandes der Verschlussvorrichtung kombiniert werden. Ergibt beispielsweise eine Abfrage einer Detektionsvorrichtung, dass die Verschlussvorrichtung oder die Verschlussvorrichtungen vollständig geschlossen sind und zeigt die Versatzerkennungsvorrichtung gleichzeitig einen Versatz zwischen der Sendespule und der Empfangsspule auf, so kann davon ausgegangen werden, dass die Tragevorrichtung unbeabsichtigt vom Körper des Patienten entfernt wurde beziehungsweise in unbeabsichtigter Weise relativ zum Körper des Patienten verschoben wurde. In diesem Fall kann ein Alarmsignal ausgegeben werden.

In weiteren Ausführungsformen kann die Tragevorrichtung verschiedene Sensoren aufweisen, durch die weitere Informationen über den Tragezustand gesammelt werden können. Zum Beispiel kann die Tragevorrichtung einen Beschleunigungssensor aufweisen, der in einer Verschlussvorrichtung und/oder einer Tragevorrichtung selbst angeordnet ist. Bei einem Beschleunigungssensor in der Verschlussvorrichtung, beispielsweise in einem Reißverschluss, kann der zeitliche Verlauf des Öffnungsvorgangs der Verschlussvorrichtung erfasst werden. Hieraus können Informationen darüber abgeleitet werden, ob die Verschlussvorrichtung beabsichtigt oder unbeabsichtigt geöffnet wurde. Ein unbeabsichtigtes Öffnen kann beispielsweise dann angenommen werden, wenn die Verschlussvorrichtung über einen sehr großen Zeitraum, beispielsweise während des Schlafs, geöffnet wurde.

Zusätzlich oder alternativ kann die Tragevorrichtung einen Schlafdetektionssensor zum Erkennen eines Schlafzustandes des Patienten aufweisen. Der Schlafzustand des Patienten kann beispielsweise über einen Lagesensor, einen Bewegungssensor und/oder einen Atemfrequenzsensor erfasst werden. Basierend auf der Information über den Schlafzustand kann zum Beispiel der Alarm auf verschiedene Weise ausgegeben werden. Beispielsweise kann die Alarmausgabe in der Nacht während des Schlafs des Patienten verzögert erfolgen, sofern ein unbeabsichtigtes Ablegen oder Verschieben der Tragevorrichtung detektiert wurde. Zunächst kann in dieser Situation das Implantat durch den implantierten Akku durch Energie versorgt werden. Möglicherweise wird während dieser Zeit durch eine erneute Bewegung des Patienten die Sendespule wieder korrekt über der Empfangsspule positioniert, so dass ein Alarm vermieden werden kann und der Patient in seinem Schlaf nicht gestört wird.

Zusätzlich oder alternativ kann die Tragevorrichtung einen Näherungssensor aufweisen. Der Näherungssensor ist bevorzugt an der Innenseite der Tragevorrichtung, das heißt zum Körper des Patienten weisend, angeordnet. Somit kann durch den Näherungssensor erfasst werden, ob die Tragevorrichtung am Körper des Patienten angelegt ist.

Zusätzlich oder alternativ kann die Tragevorrichtung einen Atmungsdetektionssensor zum Erfassen der Atmung des Patienten aufweisen. Hierbei kann es sich beispielsweise um einen Dehnungssensor handeln. Sofern erkannt wird, dass der Patient nicht oder nicht in einer bestimmten Weise atmet, kann eine entsprechender Alarm ausgegeben werden.

Weiterhin kann die erfindungsgemäße Tragevorrichtung einen Zugsensor oder einen Drucksensor aufweisen, durch den erfasst werden kann, ob die Tragevorrichtung ausreichend fest am Körper des Patienten sitzt, wobei bei einem zu lockeren Sitz ebenfalls ein Alarm ausgegeben werden kann. Ein solcher Sensor kann in der Verschlussvorrichtung oder an einer anderen Stelle der Tragevorrichtung angeordnet sein.

Die Tragevorrichtung kann weiterhin einen Sensor zur Messung des Widerstandes einer Kontaktfläche, insbesondere der menschlichen Haut, aufweisen. Durch Messung des Hautwiderstandes ist es möglich zu erkennen, ob die Tragevorrichtung gerade am Körper des Patienten angelegt ist.

Die genannten Sensoren können somit einzeln oder in Kombination miteinander verwendet werden, um Informationen über den Tragezustand und insbesondere über eine Änderung des Tragezustandes der Tragevorrichtung relativ zum Körper des Patienten zu erfassen und anschließend auszuwerten. Insbesondere ist es bevorzugt, die Informationen mehrerer Sensoren miteinander zu kombinieren und diese fusionierten Sensorinformationen auszuwerten, sodass ein Interpretieren mehrerer einzelner Handlungen oder Vorgänge möglich ist. Dies ermöglicht eine genauere Entscheidung dahingehend, ob das Ablegen der Tragevorrichtung beabsichtigt war oder nicht.

Die Tragevorrichtung kann weitere Sensoren aufweisen, durch die andere Parameter gemessen werden, die beispielsweise Auskunft über den Gesundheitszustand des Patienten geben können. Beispielsweise kann die Tragevorrichtung einen Feuchtigkeitssensor zur Messung der Feuchtigkeit einer Kontaktfläche, insbesondere der Haut des Patienten, aufweisen. Sofern die gemessene Feuchtigkeit einen definierten Schwellwert überschreitet, kann beispielsweise ein Alarm ausgegeben werden.

Sofern ein Versatz zwischen der Sende- und Empfangsspule detektiert wird, kann die Sendeleistung der Sendespule automatisch angepasst werden. Beispielsweise kann der Sendespule eine höhere Spannung zur Erzeugung eines stärkeren Magnetfeldes zugeführt werden. Alternativ oder zusätzlich kann die Magnetfeldstärke durch ein bewegliches oder in seinem Volumen oder in seiner Größe veränderliches ferromagnetisches Element an der Sende- oder Empfangsspule angepasst werden.

Wird durch einen der oben genannten Verfahrensschritte ein unbeabsichtigtes Ablegen der Tragevorrichtung erfasst, kann ebenfalls zunächst die Sendeleistung erhöht werden. Es wird geprüft ob durch dieses Erhöhen der Sendeleistung weiterhin eine ausreichende Energieübertragung erreicht werden kann. Dies kann beispielsweise der Fall sein, wenn der Patient zwar die Tragevorrichtung abgelegt hat, diese sich jedoch immer noch in der Nähe des Körpers des Patienten befindet und die Sendespule derart relativ zur Empfangsspule positioniert ist, dass immer noch Energie übertragen werden kann. Ist dies nicht mehr möglich, wird die Sendespule nicht mehr mit Spannung versorgt und ein Alarm wird ausgegeben.

Die Erfindung betrifft ferner ein Verfahren zum Erkennen des Anlagezustandes einer Tragevorrichtung zum Tragen einer Sendespule am Körper des Patienten, insbesondere einer Tragevorrichtung, wie sie bisher beschrieben wurden. Das erfindungsgemäße Verfahren umfasst die in Anspruch 6 definierten Schritte.

Weiterhin kann das Auswerten der Handlung oder Handlungen des Patienten folgende Schritte umfassen:
Es wird eine Kombination mehrerer gleichzeitiger oder aufeinader folgender Handlungen des Patienten interpretiert, die insbesondere durch mehrere Sensoren erfasst wurden. Hieraus wird eine Information darüber abgeleitet, ob das Ablegen oder Verschieben der Tragevorrichtung beabsichtigt oder unbeabsichtigt erfolgt ist.

In einer besonders bevorzugten Ausführungsform des erfindungsgemäßen Verfahrens erfolgt ein Erfassen des zeitlichen Verlaufs des Öffnungsvorgangs von mindestens einer Verschlussvorrichtung, durch die die Tragevorrichtung lösbar am Körper des Patienten befestigbar ist. Es wird von einem beabsichtigten Ablegen der Tragevorrichtung ausgegangen, wenn der erfasste Verlauf des erfassten Öffnungsvorgangs innerhalb definierter Schwellwerte einem vorgegebenen Musterverlauf entspricht. Dieser vorgegebene Musterverlauf wurde vorher im Rahmen von Versuchsmessungen ermittelt und datentechnisch in der Tragevorrichtung hinterlegt. Sofern der erfasste Verlauf des erfassten Öffnungsvorgangs nicht innerhalb definierter Schwellwerte dem vorgegebenen Musterverlauf entspricht, wird von einem unbeabsichtigten Ablegen oder Verschieben der Tragevorrichtung ausgegangen, sodass ein entsprechender Alarm ausgegeben werden kann.

Durch das erfindungsgemäße Verfahren ist es somit möglich, aufgrund verschiedener Sensorinformationen menschliche Handlungen zu erfassen und zu interpretierten, sodass hieraus genauere Informationen über den Tragezustand und insbesondere bei über eine Änderung des Tragezustandes gewonnen werden können.

In einer besonders bevorzugten Ausführungsform kann die Verschlussvorrichtung, durch die die Tragevorrichtung am Körper des Patienten befestigt wird, als ein Doppelreißverschluss ausgebildet sein. Derartige Doppelreißverschlüsse sind beispielsweise aus Sporttaschen oder Reisekoffern bekannt, wobei ein Verschließen des Reißverschlusses dadurch erfolgt, dass die beiden Reißverschlüsse aufeinander zu bewegt werden. Ein vollständiges Schließen des Reißverschlusses liegt dann vor, wenn beide Reißverschlüsse aneinander anliegen. In diesem Zustand, das heißt wenn die beiden einzelnen Reißverschlüsse aneinander anliegen, können diese durch ein Verriegelungslement, das an einem oder an beidem Reißverschlüssen ausgebildet ist, miteinander verbunden werden. Derartige Verriegelungselement werden beispielsweise in Form von Vorhängeschlössern bei Reisekoffern verwendet, die ebenfalls solche Doppelreißverschlüsse aufweisen können. Ein Öffnen des Doppelreißverschlusses ist nur möglich, wenn das Verriegelungselement entriegelt wird. Das genannte Verriegelungselement kann ferner dazu verwendet werden, eine Information darüber zu erhalten, ob die Tragevorrichtung gerade angelegt ist. Wird somit das Verriegelungselement von dem Patienten verriegelt, so steht der Tragevorrichtung die Information darüber zur Verfügung, dass die Tragevorrichtung gerade am Körper des Patienten angebracht ist. In dieser Ausführungsform muss sichergestellt werden, dass das Verriegelungselement nicht versehentlich betätigt werden kann.

In einer weiteren bevorzugten Ausführungsform der Tragevorrichtung kann die Verschlussvorrichtung redundant ausgebildet sein. Dies bedeutet, dass mehrere, insbesondere verschiedenartige, Verriegelungsvorrichtungen vorgesehen sind, durch die die Tragevorrichtung am Körper des Patienten befestigt wird. Beispielsweise kann ein Klettverschluss vorgesehen sein, der über einen Reißverschluss gelegt wird.

Insbesondere bei einem Klettverschluss als Verschlussvorrichtung kann davon ausgegangen werden, dass die Tragevorrichtung unbeabsichtigt geöffnet wurde, wenn das Öffnung des Klettverschlusses eine lange Zeit in Anspruch genommen hat. In diesem Fall wird davon ausgegangen, dass der Klettverschluss über eine unbeabsichtigte Krafteinwirkung "aufgescheuert" wurde und die Tragevorrichtung unbeabsichtigt abgelegt oder verschoben wurde. In diesem Fall kann ein Alarm ausgegeben werden.

Weiterhin ist es möglich, Detektionsvorrichtungen zum Erkennen des Verschlusszustandes der Verschlussvorrichtungen in die Verschlussvorrichtungen selbst zu integrieren. Beispielsweise können die Haken eines Verschlusses als Schalter ausgebildet sein, durch die der Schließzustand des Verschlusses erkannt werden kann.

Im Folgenden werden bevorzugte Ausführungsformen der Erfindung anhand von Figuren erläutert.

Es zeigen:
- Fig. 1: eine Frontansicht einer Ausführungsform der erfindungsgemäßen Tragevorrichtung
- Fig. 2: eine schematisierte geschnittene Seitenansicht der erfindungsgemäßen Tragevorrichtung

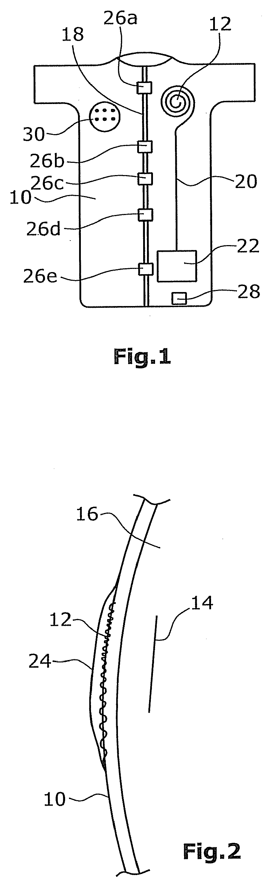

Die in Figur 1 dargestellte Tragevorrichtung 10 ist in Form einer Weste ausgebildet und kann beispielsweise über einen Reißverschluss 18 am Körper des Patienten befestigt werden. Im Brustbereich weist die Weste 10 eine Sendespule 12 auf, die beispielsweise in die Fasern der Weste 10 eingewebt sein kann. Die Sendespule 12 ist über die ebenfalls in die Weste integrierte elektrische Leitung 20 mit der an der Weste 10 angebrachten Spannungsquelle 22 verbunden.

Entlang des Reißverschlusses 18 weist die Weste 10 fünf Detektionsvorrichtungen 26 a bis 26 e auf, durch die der zeitliche Verlauf des Öffnungsvorgangs des Reißverschlusses 18 erfasst werden kann.

Weiterhin weist die Weste 10 eine Eingabevorrichtung 28 auf, bei der es sich beispielsweise um eine Taste handeln kann. Durch diese Taste kann der Patient der Tragevorrichtung 10 mitteilen, dass er diese beabsichtigt abgelegt hat oder ablegen wird.

Weiterhin weist die Weste eine Alarmvorrichtung, beispielsweise einen Lautsprecher 30 auf, durch die ein Alarmsignal ausgegeben werden kann, wenn die Weste 10 unbeabsichtigt abgelegt wurde.

Figur 2 stellt eine in die Weste 10 eingewebte Sendespule 12 näher dar. Im Brustbereich weist die Tragevorrichtung 10 eingewebte elektrisch leitende Elemente in Form von flexiblen Litzen auf, die spulenförmig gewickelt sind und somit die Sendespule 12 ausbilden. Es kann eine Konzentration des Magnetfeldes durch eine ferromagnetische Folie 24 erfolgen, die die Sendespule 12 vollständig abdeckt und an der von der Empfangsspule 14 weg weisenden Seite der Sendespule 12 angeordnet ist. Der Körper des Patienten weist das Bezugszeichen 16 auf.

## Patentansprüche

1. System mit Tragevorrichtung zum Tragen einer Sendespule (12) am Körper (16) eines Patienten, wobei die Tragevorrichtung ausgestattet ist mit einer Aufnahme- oder Befestigungsvorrichtung oder einem Aufnahmeraum zum Aufnehmen oder Befestigen der Sendespule (12) in und/oder an der Tragevorrichtung (10), und mit einer Detektionsvorrichtung (26a bis 26e) zum Erkennen eines Anlegevorgangs, eines Anlegezustandes, eines Ablegevorgangs und/oder eines Ablegezustandes der Tragevorrichtung (10) relativ zum Körper (16) des Patienten, **gekennzeichnet dadurch, dass** die Tragevorrichtung weiterhin eine Eingabevorrichtung (28) zum Eingeben einer Information über ein beabsichtigtes Ablegen der Tragevorrichtung (10) durch den Benutzer in die Tragevorrichtung (10) aufweist, wobei die Tragevorrichtung (10) weiterhin eine Alarmvorrichtung (30) zum Ausgeben eines Alarmsignals bei einem unbeabsichtigten Ablegen oder Verschieben der Tragevorrichtung (10) aufweist oder mit einer solchen, die Bestandteil des Systems ist, datentechnisch verbindbar ist, wobei durch die Alarmvorrichtung (30) kein Ausgeben eines Alarmsignals erfolgt, sofern durch den Benutzer an der Eingabevorrichtung (28) die Eingabe einer Information über ein beabsichtigtes Ablegen der Tragevorrichtung (10) erfolgt ist.

2. System nach Anspruch 1, **gekennzeichnet durch**, mindestens eine Verschlussvorrichtung (18) zum lösbaren Befestigen der Tragevorrichtung (10) am Körper (16) des Patienten, wobei die Detektionsvorrichtung (26a bis 26e) in oder an der Verschlussvorrichtung (18) derart ausgebildet ist, dass **durch** die Detektionsvorrichtung (26a bis 26e) ein Öffnen der Verschlussvorrichtung (18) und bei mehreren Verschlussvorrichtungen insbesondere der zeitliche Verlauf des Öffnungsvorgangs der mehreren Verschlussvorrichtungen erfassbar ist.

3. System nach einem der Ansprüche 1 oder 2, **dadurch gekennzeichnet, dass** die Eingabevorrichtung (28) eine Spracheingabevorrichtung oder mindestens eine Taste umfasst.

4. System nach einem der Ansprüche 1 bis 3, **gekennzeichnet durch** eine Versatzerkennungsvorrichtung zum Erkennen eines axialen und/oder radialen Versatzes zwischen der Sendespule (12) in oder an der Tragevorrichtung (10) und einer im Körper (16) des Patienten implantierten Empfangsspule (14), wobei **durch** eine Alarmvorrichtung (30) bei einem Versatz, der einen definierten Schwellwert überschreitet, ein Ausgeben eines Alarmsignals erfolgt, sofern **durch** eine Eingabevorrichtung (28) kein Eingeben einer Information über ein beabsichtigtes Ablegen der Tragevorrichtung (10) durch den Patienten erfolgt ist.

5. System nach einem der Ansprüche 1 bis 4, **dadurch gekennzeichnet, dass** die Tragevorrichtung (10) einen Beschleunigungssensor aufweist, der in einer Verschlussvorrichtung (18) und/oder in der Tragevorrichtung (10) selbst angeordnet ist, und/oder die Tragevorrichtung (10) einen Schlafdetektionssensor zum Erkennen eines Schlafzustandes des Patienten aufweist, und/oder die Tragevorrichtung (10) einen Näherungssensor aufweist, und/oder die Tragevorrichtung (10) einem Atmungsdetektionssensor zum Erfassen der Atmung des Patienten aufweist.

6. Verfahren zum Erkennen des Anlagezustandes einer Tragevorrichtung (10) zum Tragen einer Sendespule (12) am Körper (16) eines Patienten, wobei das Verfahren die folgenden Schritte aufweist: Erfassen einer beabsichtigten oder unbeabsichtigten Handlung, einer zeitlichen Abfolge von beabsichtigten und/oder unbeabsichtigten Handlungen und/oder mehrere gleichzeitig stattfindende beabsichtigte und/oder unbeabsichtigte Handlungen des Patienten, die ein Ablegen der Tragevorrichtung (10) oder ein Verschieben der Tragevorrichtung (10) relativ zum Körper (16) des Patienten zur Folge haben, Auswerten der erfassten Handlung und/oder Handlungen des Patienten und anschließendes Entscheiden, ob der Patient die Tragevorrichtung (10) beabsichtigt oder unbeabsichtigt abgelegt und/oder verschoben hat, Ausgeben eines Alarmsignals bei einem unbeabsichtigten Ablegen und/oder Verschieben der Tragevorrichtung (10), **dadurch gekennzeichnet, dass** das Auswerten der Handlung oder Handlungen des Patienten folgende Schritte aufweist:
Abfragen, ob der Patient an einer Eingabevorrichtung (28) an der Tragevorrichtung (10) eine Information über ein beabsichtigtes Ablegen der Tragevorrichtung (10) eingegeben hat, wobei in diesem Fall kein Alarmsignal ausgegeben wird.

7. Verfahren nach Anspruch 6, **dadurch gekennzeichnet, dass** das Auswerten der Handlung oder Handlungen des Patienten folgende Schritte umfasst:
Interpretieren einer Kombination mehrerer gleichzeitiger oder aufeinander folgender Handlungen des Patienten, die insbesondere durch mehrere Sensoren erfasst wurden, zum Ableiten der Information, ob das Ablegen oder Verschieben der Tragevorrichtung (10) beabsichtigt oder unbeabsichtigt erfolgt ist.

8. Verfahren nach einem der Ansprüche 6 bis 7, **gekennzeichnet durch** die Schritte:
Erfassen des zeitlichen Verlaufs des Öffnungsvorgangs von mindestens einer Verschlussvorrichtung (18), **durch** die die Tragevorrichtung (10) lösbar am Körper (16) des Patienten befestigbar ist,
wobei von einem beabsichtigten Ablegen der Tragevorrichtung (10) ausgegangen wird, wenn der erfasste Verlauf des erfassten Öffnungsvorgangs innerhalb definierter Schwellwerte einem vorgegebenen Musterverlauf entspricht.
und andernfalls von einem unbeabsichtigten Ablegen oder Verschieben der Tragevorrichtung (10) ausgegangen wird, sofern der erfasste Verlauf des erfassten Öffnungsvorgangs nicht innerhalb definierter Schwellwerte einem vorgegebenen Musterverlauf entspricht.

## Claims

1. A system comprising a support device for supporting a transmission coil (12) on the body (16) of a patient, the support device being equipped with
a receiving or securing device or a receiving space for receiving or securing the transmission coil (12) in and/or on the support device (10), and with
a detection device (26a to 26e) for detecting a process of putting on the support device, a state of wearing, a process of removal and/or a state of removal of the support device (10) relative to the body (16) of the patient,
**characterized in that** the support device further comprises
an input device (28) for the input of information about an intentional shedding of the support device (10) by the user into the support device (10),
wherein the support device (10) further comprises an alarm device (30) for emitting an alarm signal upon an unintentional shedding or shifting of the support device (10), or can be connected with such an alarm device by a data link, wherein the alarm device (30) emits no alarm signal, if the user has input information at the input device (28) indicating an intentional shedding of the support device (10).

2. The system of claim 1, **characterized by** at least one closure device (18) for the releasable securing of the support device (10) on the body (16) of the patient, wherein the detection device (26a to 26e) is configured in or at the closure device (18) such that the detection device (26a to 26e) can detect the opening of the closure device (18) and, in case of a plurality of closure devices, can detect in particular the temporal course of the opening of the plurality of closure devices.

3. The system of one of claims 1 or 2, **characterized in that** the input device (28) in particular comprises a voice input device or at least a key.

4. The system of one of claims 1 to 3, **characterized by** an offset detection device for detecting an axial and/or radial offset between the transmission coil (12) in or on the support device (10) and a receiving coil (14) implanted in the body (16) of a patient,
wherein in case of an offset exceeding a defined threshold value, an alarm device (30) emits an alarm signal, if the patient has not input information at the input device (28) indicating an intentional shedding of the support device (10).

5. The system of one of claims 1 to 4, **characterized in that** the support device (10) comprises an acceleration sensor arranged in a closure device (18) and/or in the support device (10) itself,
and/or the support device (10) comprises a sleep detection sensor for detecting a sleeping state of a patient,
and/or the support device (10) comprises a proximity sensor,
and/or the support device (10) comprises a breathing sensor for detecting the breathing of a'patient.

6. A method for detecting the wearing state of a support device (10) for supporting a transmission coil (12) on the body (16) of a patient, the method comprising the following steps:
detecting an intentional or unintentional action, a chronological sequence of intentional and/or unintentional actions and/or a plurality of simultaneous intentional and/or unintentional actions of a patient that result in a shedding of the support device (10) or a shifting of the support device (10) relative to the body (16) of a patient,
evaluating the detected action and/or actions of a patient and, subsequently, deciding whether a patient has shed and/or shifted the support device (10) intentionally or unintentionally,
emitting an alarm signal in the case of an unintentional shedding and/or shifting of the support device (10),
**characterized in that**
the evaluation of the action or the actions of a patient comprises the following steps:
inquire, whether a patient has input information into an input device (28) on the support device (10), indicating an intentional shedding of the support device (10), in which case no alarm signal is emitted.

7. The method of claim 6, **characterized in that** the evaluation of the action or the actions of a patient comprises the following steps:
interpreting a combination of a plurality of simultaneous or successive actions of a patient, which have been detected in particular by a plurality of sensors, for deriving information about whether the shedding or shifting of the support device (10) was intentional or unintentional.

8. The method of one of claims 6 to 7, **characterized by** the following steps:
detecting the temporal course of the opening process of at least one closure device (18) by which the support device (10) can be releasably fastened on the body (16) of a patient,
wherein an intentional shedding of the support device (10) is assumed, it the detected progress of the opening process corresponds to a predefined reference progress within defined threshold values,
and otherwise, an unintentional shedding or shifting of the support device (10) is assumed, if the detected progress of the detected opening process does not correspond to a predefined reference progress within defined threshold values.

## Revendications

1. Système comprenant
un dispositif de support servant à supporter une bobine émettrice (12) au niveau du corps (16) d'un patient, le dispositif de support étant équipé
d'un dispositif de réception ou de fixation ou d'un espace de réception destiné à recevoir ou monter la bobine émettrice (12) dans et/ou sur le dispositif de support (10),
et
d'un dispositif de détection (26a à 26e) servant à détecter un processus de pose, un état de pose, un processus de retrait et/ou un état de retrait du dispositif de support (10) par rapport au corps (16) du patient, **caractérisé en ce que** le dispositif de support comprend en outre un dispositif d'entrée (28) servant à entrer dans le dispositif de support (10) une information sur un retrait volontaire du dispositif de support (10) par l'utilisateur,
le dispositif de support (10) comportant en outre un dispositif d'alarme (30) servant à délivrer un signal d'alarme en cas de retrait ou de déplacement involontaire du dispositif de support (10) ou pouvant être relié par une technique informatique à un tel dispositif d'alarme,
le dispositif d'alarme (30) ne délivrant aucun signal d'alarme si l'entrée d'une information relative à un retrait volontaire du dispositif de support (10) est effectuée par l'utilisateur au niveau du dispositif d'entrée (28).

2. Système selon la revendication 1, **caractérisé par** au moins un dispositif de fermeture (18) servant à fixer de manière amovible le dispositif de support (10) au corps (16) du patient, le dispositif de détection (26a à 26e) dans ou sur le dispositif de fermeture (18) étant conçu de telle sorte que le dispositif de détection (26a à 26e) peut détecter une ouverture du dispositif de fermeture et, en cas de pluralité de dispositifs de fermeture, en particulier le déroulement dans le temps du processus d'ouverture de la pluralité de dispositifs de fermeture.

3. Système selon l'une des revendications 1 ou 2, **caractérisé en ce que** le dispositif d'entrée (28) comporte un dispositif d'entrée vocale ou au moins une touche.

4. Système selon l'une des revendications 1 à 3, **caractérisé par** un dispositif de détection de décalage servant à détecter un décalage axial et/ou radial entre la bobine émettrice (12) dans ou sur le dispositif de support (10) et une bobine de réception implantée dans le corps (16) du patient (14)
un signal d'alarme étant délivré par un dispositif d'alarme (30) en cas de décalage supérieur à une valeur de seuil définie si aucune entrée d'une information relative à un retrait volontaire du dispositif de support (10) n'est effectuée par le patient par le biais d'un dispositif d'entrée (28).

5. Système selon l'une des revendications 1 à 4, **caractérisé en ce que** le dispositif de support (10) comporte un capteur d'accélération qui est disposé dans un dispositif de fermeture (18) et/ou dans le dispositif de support (10) lui-même, et/ou le dispositif de support (10) comporte un capteur de détection de sommeil servant à détecter un état somnolent du patient, et/ou le dispositif de support (10) comporte un capteur de proximité, et/ou le dispositif de support (10) comporte un capteur de détection de respiration servant à détecter la respiration du patient.

6. Procédé de détection de l'état d'installation d'un dispositif de support (10) servant à supporter une bobine émettrice (12) au niveau du corps (16) d'un patient, le procédé comportant les étapes suivantes consistant à :
détecter une action volontaire ou involontaire, une séquence temporelle d'actions volontaires et/ou involontaires et/ou plusieurs actions simultanées volontaires et/ou involontaires du patient, qui ont pour conséquence un retrait du dispositif de support (10) ou un déplacement du dispositif de support (10) par rapport au corps (16) du patient,
évaluer l'action détectée et/ou les actions détectées du patient puis décider si le patient a retiré et/ou déplacé volontairement ou involontairement le dispositif de support (10),
délivrer un signal d'alarme en cas de retrait et/ou de déplacement involontaire du dispositif de support (10), **caractérisé en ce que**
l'évaluation de l'action ou des actions du patient comporte les étapes suivantes consistant à :
demander si le patient a entré au niveau d'un dispositif d'entrée (28) sur le dispositif de support (10) une information relative à un retrait volontaire du dispositif de support (10), aucun signal d'alarme n'étant émis dans ce cas.

7. Procédé selon la revendication 6, **caractérisé en ce que** l'évaluation de l'action ou des actions du patient comporte les étapes suivantes consistant à :
Interpréter une combinaison de plusieurs actions simultanées ou successives des patients qui ont été détectées notamment par plusieurs capteurs pour obtenir l'information si le retrait ou le déplacement du dispositif de support (10) a été effectué volontairement ou involontairement.

8. Procédé selon l'une des revendications 6 à 7, **caractérisé par** les étapes consistant à :
détecter le déroulement dans le temps du processus d'ouverture d'au moins un dispositif de fermeture (18) par le biais duquel le dispositif de support (10) peut être fixé de façon amovible sur le corps (16) du patient,
un retrait volontaire du dispositif de support (10) étant supposé si le déroulement détecté du processus d'ouverture correspond à un modèle de déroulement prédéterminé entre des valeurs de seuil définis,
sinon un retrait ou déplacement involontaire du dispositif de support (10) est supposé si le déroulement détecté du processus d'ouverture ne correspond pas, à un modèle de déroulement prédéterminé entre des valeurs de seuil définis.
